# EUROPEAN PATENT SPECIFICATION

(11) **EP 2 370 011 B1**
(45) Date of publication and mention of the grant of the patent: **10.04.2013**
(21) Application number: 09820276.5
(22) Date of filing: 29.09.2009
(51) Int. Cl.: A61B 17/70

(54) **A SPINAL CONSTRUCTION ASSEMBLY COMPRISING AN INTERCONNECTING DEVICE**
WIRBELSÄULENVERBINDUNGSVORRICHTUNG UND STABILISIERUNGSSYSTEM
SYSTÈME STABILISATEUR UTILISANT UN DISPOSITIF D'INTERCONNEXION SPINALE

(30) Priority: 15.10.2008 EP 08305684
(43) Date of publication of application: 05.10.2011
(73) Proprietor: ZIMMER SPINE, 33000 Bordeaux (FR)
(72) Inventor: JOUVE, Jean-Luc, F-13385 Marseille Cedex 5 (FR); MINFELDE, Richard, F-75019 Paris (FR); MAZDA, Keyvan, F-75020 Paris (FR)
(74) Representative: Besnard, Christophe Laurent
(86) International application number: PCT/EP2009/062630
(87) International publication number: WO 2010/043496

(56) References cited:
- WO-A-97/15231
- WO-A-02/076315
- FR-A- 2 820 968
- FR-A- 2 849 590
- US-A1- 2007 239 159

## Description

### FIELD OF THE INVENTION

The present invention relates to a stabilizing system according to claim 1. It comprises a device for interconnecting a connecting rod with a separate rod and fixing these rods to a bone.

More particularly, it relates to a device for interconnecting a spinal construction assembly comprising a connecting rod with a separate rod and fixing them to a vertebra, and a stabilizing system including such an interconnecting device.

One field of application for the invention is holding bones in a relative position, for example, to aid in the healing of breaks or the positioning of bones, or the treatment of scoliosis, or otherwise to correct abnormal curvatures of the spine.

### BACKGROUND OF THE INVENTION

The spine is formed of superposed vertebrae, normally aligned along a vertebral axis from the lumbar vertebrae to the cervical vertebrae, each having a posterior wall from which projects a spinous process and two lateral edges from the walls of which project ribs and/or transverse process. Each vertebra also has two lateral pedicles and lamina surfaces.

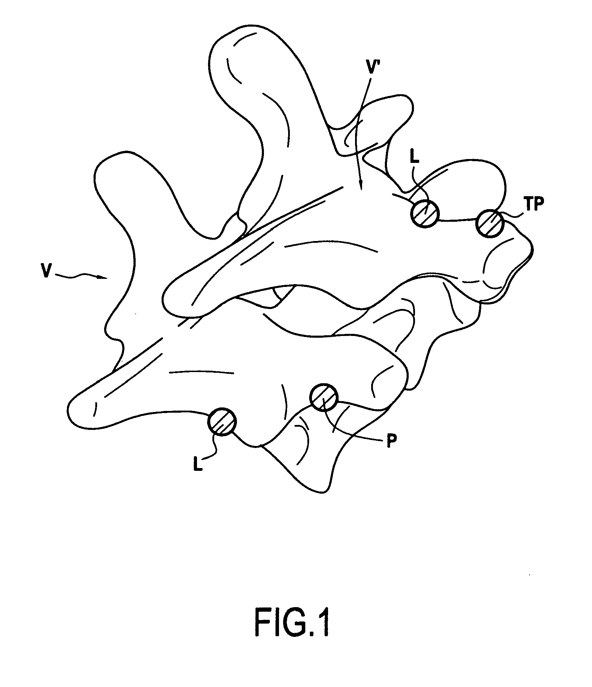

Accompanying figure 1 shows vertebrae V and V' with the different parts thereof. Reference TP designates the transversal processes, reference P designates the pedicles of the vertebra and reference L designates the lamina.

In order to straighten or stabilize the vertebrae of a spine it is well known to use a stabilizing system which includes a longitudinal connecting rod and several fixing elements. Each fixing element is secured to one of the vertebrae to be stabilized and the connecting rod is secured to each fixing element, so that portions of the connecting rod are secured to the vertebrae to be stabilized. The fixing elements include a head to be secured to the rod and a fixing member which may consist of a screw (for example a pedicle screw) or a hook. When the patient who is equipped with such a stabilizing system moves his spine, forces are developed by the fixing members of the fixing elements in the vertebrae. In particular, the fixing elements disposed at the extremities of the rod of the stabilizing system or spinal construction assembly, develop the greatest forces. It is therefore apparent that it would be advantageous to try to decrease the forces developed at the extremities of the spinal construction assembly.

Moreover, autostable claw systems are also well known. Such a system is described, for example, in EP 0 571 619. This system includes two fixing elements, each one being provided with a hook member, and a rod to interconnect the fixing elements and, consequently, the vertebrae on which the fixing elements are secured. When defining with the rod the appropriate distance between the two fixing elements, an appropriate stabilization of the vertebrae is obtained.

Document WO9715231A discloses a stabilizing system according to the preamble of claim 1.

In another use of the autostable claw system, the hook members of the autostable claw system can be secured to different parts of the same vertebra.

### BRIEF SUMMARY OF THE INVENTION

A first goal is to provide an interconnecting device which is more easily implantable by the physician than the already known devices, such a device being intended, in particular, for interconnecting a spinal construction assembly, more particularly the extremity of said assembly, with a separate rod, said separate rod being preferably, but not necessarily, part of an autostable claw system.

To achieve this goal, according to the one embodiment of the present disclosure, there is provided a stabilizing system comprising a device for interconnecting a connecting rod with a separate rod and fixing them to a bone, this device comprising a connecting body and a hook member for securing said connecting body to said bone, said hook member being fixed to said connecting body, said connecting body comprising a first connecting member and a second connecting member, said first connecting member being adapted to receive a connecting rod, said second connecting member being adapted to receive a first end of a separate rod so that the direction of the separate rod is adaptable with respect to the direction of the connecting rod.

Said connecting rod is part of a spinal construction assembly, and said bone is a vertebra. Accordingly, the spinal construction system assembly and, in particular, the extremity of this assembly, is connected to one end of the separate rod and fixed to the vertebra thanks to the interconnecting device, which is a unique device.

Due to the fact that the second connecting member allows the separate rod to have an adaptable direction with respect to the direction of the connecting rod, the surgeon (or other operative) can choose freely the part of the vertebra to which the interconnecting device is fixed.

According to the invention, there is provided a device for interconnecting a connecting rod with a separate rod and fixing these rods to a vertebra, this device being such that it comprises a connecting body, and a hook member for securing the connecting body to a bone, the hook member being fixed to the connecting body; such that said connecting body comprises a first connecting member, and a second connecting member; and such that said first connecting member has a slot adapted to receive a connecting rod, said slot opening into two opposite side faces of the first connecting member, so that said slot may receive the connecting rod from both sides of the first connecting member, and such that said second connecting member is adapted to receive a first end of a separate rod, in a manner that the direction of the separate rod is adaptable with respect to the direction of the connecting rod.

The interconnecting device may be used for interconnecting a free end of a connecting rod with a separate rod, the slot of said first connecting member being adapted to receive the free end of the connecting rod.

According to an embodiment, the second connecting member is provided with an opening which opens into at least one side face of the second connecting member, said opening forming a socket part of a ball-and-socket joint and being configured to receive a ball end of a separate rod. Said ball-and-socket joint can also be called "rotulating assembly".

According to an embodiment, said opening has a partially spherical bottom wall delimiting said socket part.

According to an embodiment, the first end of the separate rod is provided with a spherical member which forms the ball member, or male part, of said ball-and-socket joint, and the second connecting member is provided with an opening which terminates into, i.e. opens into, a side face of the second connecting member, this opening having a partially spherical bottom wall which forms the socket part, or female part, of said ball-and-socket joint, said ball member being adapted to be engaged within said socket part.

According to an embodiment, said hook member and said connecting body have a common median plane, said first connecting member having an axis and overlapping said hook member, said second connecting member having an axis contained in said median plane and substantially parallel to the axis of the first connecting member and being offset with respect to said first connecting member in a direction perpendicular to the axis of said first connecting member.

Because the second connecting member is offset with respect to the first connecting member in a direction which is perpendicular to the direction of the axis of the connecting member, the thickness of the head of the interconnecting device is not increased.

The hook member may overlap the first connecting member or the second connecting member in a direction which is substantially parallel to the axis of the first connecting member.

According to an embodiment, the first connecting member overlaps the hook member in a direction which is substantially parallel to the axis of the first connecting member.

According to an embodiment, the opening of the second connecting member opens into two opposite side faces of the second connecting member, so that said opening is adapted to receive the first end of a separate rod from both sides of the second connecting member, i.e. from both sides with respect to said median plane.

Another objective is to provide a stabilizing system with an interconnecting device such as described above.

Thus, there is provided a stabilizing system comprising a spinal construction assembly, a separate rod having first and second ends, an interconnecting device such as described above for interconnecting said spinal construction assembly and the first end of said separate rod.

According to one embodiment of the disclosure, there is provided a stabilizing system for stabilizing vertebrae comprising:
- a spinal construction assembly comprising a connecting rod;
- a separate rod having first and second ends; and
- an interconnecting device such as described above, for interconnecting said connecting rod with the first end of said separate rod, the connecting body of the interconnecting device being intended to be secured to a vertebra.

The spinal construction assembly comprises a plurality of fixing devices to secure portions of the connecting rod with the vertebrae to be stabilized.

The stabilizing system further comprises a fixing element for fixing said separate rod to a vertebra and, more particularly, for fixing the second end of said separate rod to a vertebra.

It should be noted that the stabilizing system is basically a combination of a spinal construction system and of an autostable claw system, this autostable claw system being formed by the second connecting member of the interconnecting device, the separate rod, and the fixing element. The purpose of the autostable claw system is to obtain a decrease in the forces developed within the vertebra by the extremity of the spinal construction system.

According to an embodiment, the separate rod is designed so that said interconnecting device and said fixing element are mounted on the same vertebra.

According to another embodiment, the separate rod is designed so that said interconnecting device and said fixing element are mounted on two adjacent vertebrae.

As the separate rod can have an adaptable direction with respect to the direction of the rod of the spinal construction, the surgeon can freely choose the part of the vertebra to which the interconnecting device is fixed and the part of the vertebra to which the fixing element is secured. This part can be the transverse process, the pedicle or the lamina. The hook member of the interconnecting device and the fixing member (which may be another hook member) of the fixing element can be fixed to two different parts of the same vertebra or to two adjacent different vertebrae.

In both cases, the rods of the spinal construction and of the separate rod can form an angle of less than 90 degrees. This means that the separate rod "returns" towards the rod of the spinal construction.

In both cases, the angle between the two rods can also be more than 90 degrees. This means that the separate rod extends "beyond" the rod of the spinal construction.

### BRIEF DESCRIPTION OF THE DRAWINGS

The accompanying drawings are not necessarily to scale, emphasis instead generally being placed upon illustrating the principles of the invention.
Figure 1, already described, is a perspective view of two vertebrae showing the different parts of the vertebrae.
Figure 2 is a perspective view of an example of interconnecting device according to the present disclosure.
Figure 3 is a sectional view of the interconnecting device according to plane P of figure 2.
Figure 4 is an exploded view showing the combination of a spinal construction assembly with a separate rod, the interconnecting device being provided with a pedicle hook.
Figure 5 is similar to figure 4 but the interconnecting device is provided with a lamina hook.

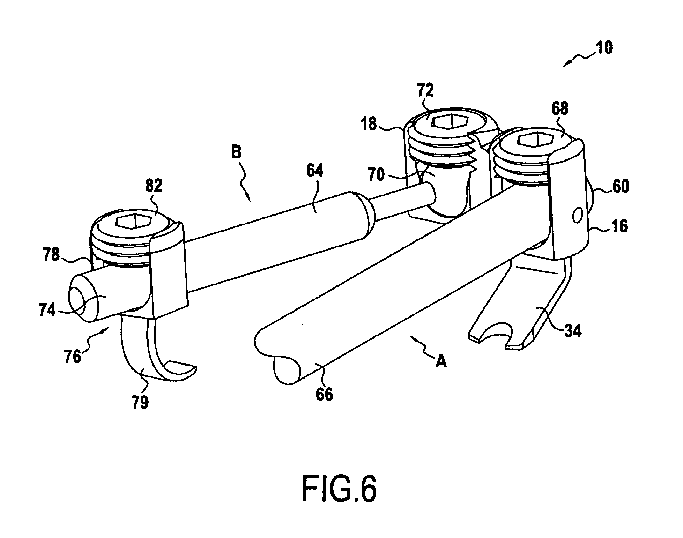

Figure 6 is similar to figure 4 but with the components assembled.
Figures 7A to 7D are schematic drawings which show four different combinations of a spinal construction assembly with an autostable claw system.

### DETAILED DESCRIPTION OF THE INVENTION

In the following detailed description, it is referred to the accompanying drawings showing examples of interconnecting device and stabilizing system according to the present disclosure. It is intended that these examples are to be considered only as illustrations of the invention. The invention is not limited to these examples.

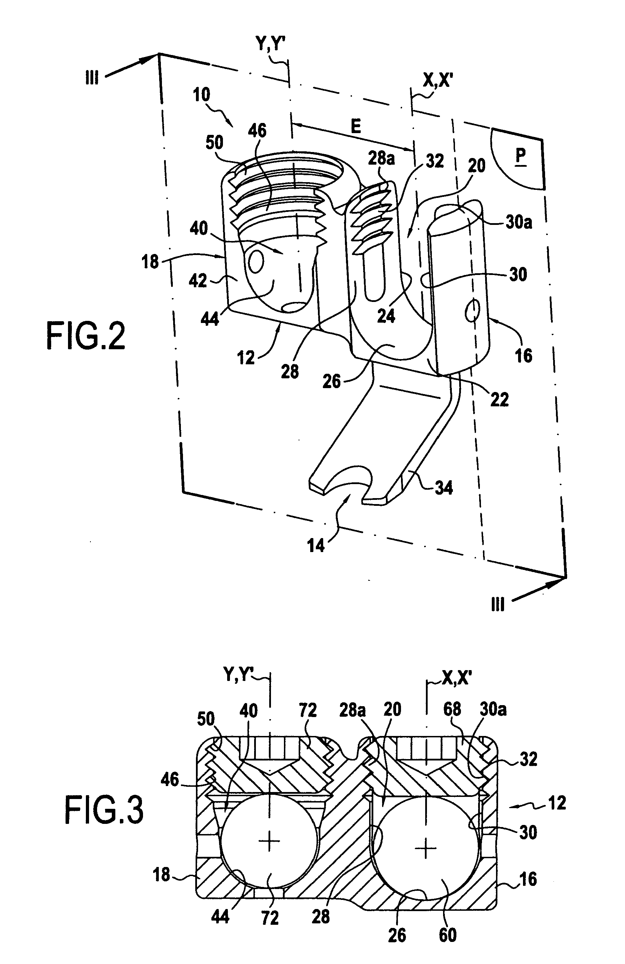

The interconnecting device 10 of figures 2 and 3, comprises a connecting body 12 and a hook member 14.

The connecting body 12 includes a first connecting member 16 and a second connecting member 18. Preferably, the first connecting member 16, the second connecting member 18 and the hook member 14 are formed as a single piece.

Preferably, the first connecting member 16, which forms a connecting head, is provided with an opening which goes through the connecting head and terminates into, or opens into, two opposite faces of the head. In the example of figures 2 and 3, said opening is a slot 20 which opens into two opposite side faces 22 and 24 of the head. Thus, the slot 20 forms a passage going from a first side face 22 to a second side face 24 (which is opposite to the first one) of the first connecting member 16. The slot 20 also opens into the upper end face of the first connecting member 16. The slot 20 is defined between a bottom wall 26 which is substantially semi-cylindrical and two lateral walls 28 and 30 which are substantially straight. The lateral walls 28, 30 extend parallel to the longitudinal axis XX' of the first connecting member 16.

The upper portion 28a and 30a of the lateral walls 28 and 30 defines portions of cylindrical surfaces. These upper portions 28a and 30a are provided with a threading 32. In this example, the threading 32 is internal, but one skilled in the art will appreciate that it might be external.

Preferably, the hook member 14 is formed by a hook 34. In the embodiment shown in figure 2, the hook member 14 is a pedicle hook. However, the hook member 14 may be a lamina hook. The hook 34 is disposed below the first connecting head 16.

Preferably, the second connecting member 18 is provided with an opening 40 which terminates into, or opens into, the side face 42 of the second connecting member 18. The opening 40 also opens into the upper end face of the second connecting member 18. The bottom 44 of the opening 40 is defined by a wall having substantially the shape of a portion of a half spherical surface. As will be explained hereinafter, the partly spherical bottom 44 of the opening 40 delimits the female part 48, or socket part, of a ball-and-socket joint.

The upper part 46 of the opening 40 is formed by a wall 48 having the shape of a portion of a cylindrical surface and extend parallel to the longitudinal axis YY' of the second connecting member 18. The upper part 46 is provided with an internal threading 50. One skilled in the art will appreciate that threading 50 might be external.

The first connecting member 16 has a longitudinal axis XX' and the second connecting member 18 has a longitudinal axis YY'. These two axes are parallel one with the other and are disposed on a plane P. The plane P forms a median plane for the interconnecting device 10. The first and second connecting members 16 and 18 are placed side by side in the direction perpendicular to the direction of the axes XX' and YY'. As a result, the axes XX' and YY' are offset by a length E in the plane P.

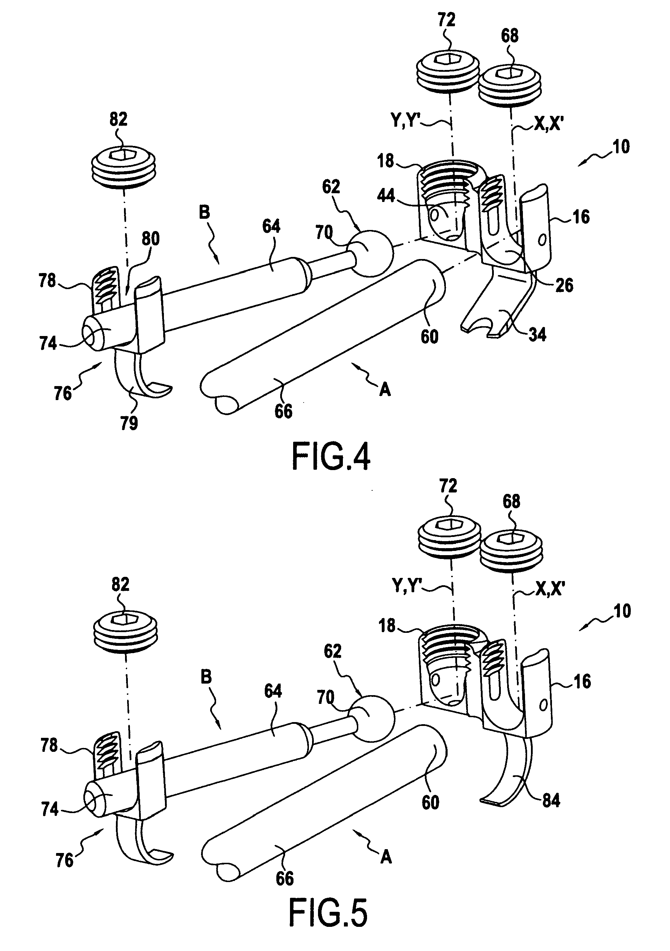

Referring now to figure 4, the interconnecting device 10 is used to form a spinal stabilizing system which combines a spinal construction system A, or spinal construction assembly, and an adjustable and autostable claw system B. The function of the device 10 is to interconnect the extremity 60 of the spinal construction system A with an end 62 of a separate rod 64 which forms part of the autostable claw system B.

In figure 4, the spinal construction system A is symbolically represented by a longitudinal connecting rod 66 having a free end 60. As is well known, the entire spinal construction A comprises the connecting rod 66 and a plurality of fixing devices (not shown in the figure) to secure intermediate portions of the rod 66 with the vertebrae to be stabilized by the spinal construction system A.

The free end 60 of the rod 66 of the spinal construction system A is adapted to be engaged into the opening 20 of the first connecting member 16 of the interconnecting device 10. For this purpose, the diameter of the bottom wall 26 of the opening 20 is slightly greater than the diameter D of the rod 66. When the end 60 of the rod 66 is engaged into the slot 20, this end 60 is connected to the connecting member by means of a screw 68 which cooperates with the threading 32, the rod 66 being clamped between the screw 68 and the bottom wall 26 of the opening 20.

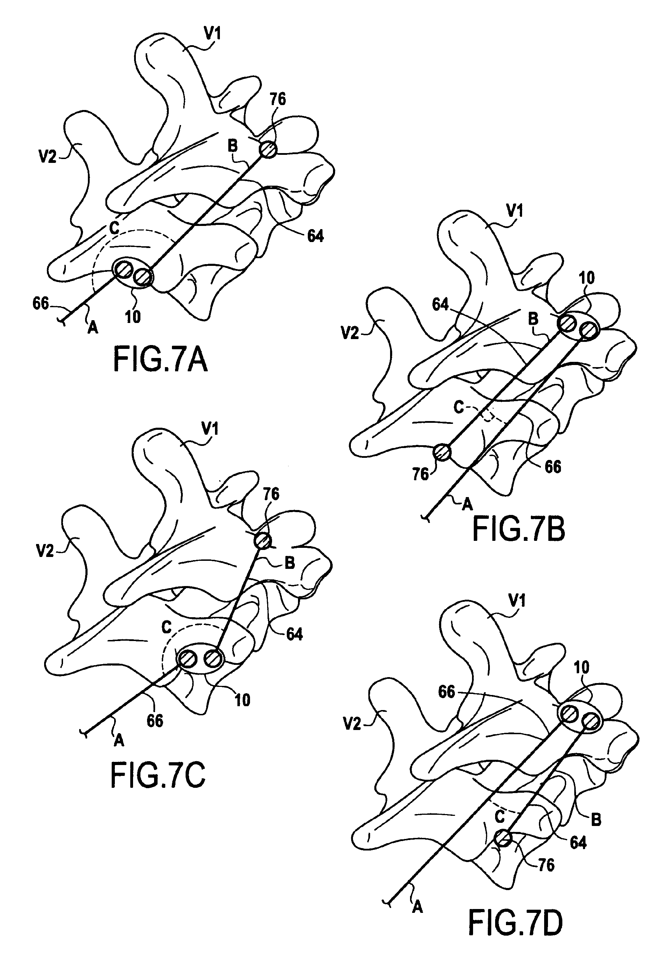

The opening 20 may receive the connecting rod 66 from both sides of the connecting member 16. Thus, it makes the positioning of the interconnecting device 10 with respect to the connecting rod 66 easier. In particular, it is possible to cause the interconnecting device 10 to slip onto the connecting rod 66 (before the locking step) and, thus, the connecting member 16 may be positioned more or less close to the tip of the connecting rod 66. Moreover, different layouts may be contemplated when implanting the device, as illustrated in FIGS 7A-7D and as described hereinafter. The device is, therefore, easily implantable by the physician or other operative.

As shown in figure 4, the end 62 of the separate rod 64 is provided with a spherical member 70 which forms the male part, or ball member, of a ball-and-socket joint and is adapted to be engaged within the partially spherical female part 48 of the opening 40 of the second connecting member 18 forming the socket part of the ball-and-socket joint. The spherical member 70 can be connected to the connecting member 18 by means of a screw 72 which can cooperate with the threading 50 of the opening of connecting member 18, the spherical member 70 being clamped between the screw 72 and the bottom 44 of the opening 40.

The second end 74 of the separate rod 64 can be connected to a fixing element 76. Preferably, the fixing element 76 comprises a connecting head 78 provided with a slot 80 to receive the rod 64 and a hook 79 to fix the fixing element 76 to a vertebra. It is therefore possible to cause the fixing element 76 to slip onto the separated rod 64 (before the locking step) and the fixing element 76 may be positioned more or less close to the tip of the separated rod 64. Thus, the length between the fixing element 76 and the second connecting member 18 is adjustable and the device is easily implantable by the physician or other operative.

In the example shown in figure 4, the hook 79 is a lamina hook.

The second end 74 of the rod 64 can be secured to the connecting head 78 by means of a screw 82.

In figure 5, the interconnecting device 10 is provided with a lamina hook 84.

In the figures, the hook member 34 or 84 has the same axis XX' as, i.e. is "aligned" with, the first connecting member 16. However, it may be aligned with the second connecting member 18.

A preferred use of the interconnecting device 10 is to interconnect an autostable claw system B and a spinal construction system A.

The autostable claw system B comprises the rod 64, the fixing element 76 with its hook 79, and the second connecting member 18 and the hook 34 of the interconnecting device 10.

As already explained, the connection between the end 62, 70 of the separate rod 64 and the second connecting member 18 is achieved by a ball-and-socket joint consisting in the spherical member 70 and the partially spherical bottom wall 44 of the connecting member 18. As a result, the direction of the separate rod 64 can be chosen by the surgeon, or other operative, before screwing the screw 72 with the connecting member 18. Thus, the surgeon can freely choose the part of the vertebra to which the fixing element 76 is fixed. This feature significantly simplifies the action of the surgeon as will be explained below with reference to figures 7A and 7D.

Moreover, the two connecting members 16 and 18 are disposed side by side. Consequently, the interconnecting device is less aggressive than a device where the two connecting members are superposed.

Figures 7A to 7D illustrate the great number of possible positions of the autostable claw system B and the spinal construction system A. These figures also illustrate the great number of possible fixing locations of the stabilizing system to the vertebrae.

In the case of figure 7A, the interconnecting device 10 and the fixing element 76 are both fixed to a lamina of vertebrae V1 and V2, and the autostable claw system B extends beyond the spinal construction system A. The angle C between the rods of the systems A and B is substantially equal to 180 degrees.

In the case of figure 7B, the interconnecting device 10 and the fixing element 76 are still secured to a lamina of vertebrae V1 and V2 but the angle C between the autostable claw system B and the spinal construction system A is greatly reduced. The rods of the systems A and B are substantially parallel and the system B "returns" towards the system A.

In the case of figure 7C, the fixing element 76 is fixed to a lamina of vertebra V1 and the interconnecting device 10 is fixed to a pedicle of vertebra V2 and the angle C is approximately 150 degrees.

Finally, in the case of figure 7D, the interconnecting device 10 is fixed to a lamina of vertebra V1 and the fixing element 76 is secured to a pedicle of vertebra V2. The angle C is about 10 degrees.

In the examples of figures 7A to 7D, the fixing element 76 and the interconnecting device 10 are fixed to two different vertebrae. However, they may be fixed to the same vertebra.

The separate rod 64 of system B is smaller in length than the connecting rod 66 of system A and said separate rod 64 is designed so that said interconnecting device 10 and said fixing element 76 are mounted on the same vertebra or on two adjacent vertebrae. The connecting rod 66 is designed so that more than two vertebrae are stabilized by the spinal construction system A.

In the examples of figures 7A to 7D, the separate rod 64 has a straight shape and a desired length so that said interconnecting device 10 and said fixing element 76 are mounted on two adjacent vertebrae.

In another embodiment (not shown), said interconnecting device 10 and said fixing element 76 are fixed to two different parts of the same vertebra. In this case, the rod 64 of the autostable claw system B has a reduced length.

In both cases, the anchoring of the extremity of the spinal construction system A into the vertebra is improved by the provision of the autostable claw system B.

## Claims

1. A stabilizing system for stabilizing vertebrae comprising:
- a spinal construction assembly comprising a connecting rod (66) and a plurality of fixing devices to secure portions of the connecting rod (66) with the vertebrae to be stabilized;
the stabilizing system further comprising:
- a separate rod (64) having first and second ends (62, 74);
and
- an interconnecting device (10) for interconnecting said connecting rod (66) with the first end (62) of said separate rod (64) and fixing these rods (64, 66) to a vertebra, this interconnecting device comprising:
- a connecting body (12), and
- a hook member (14) for securing said connecting body (12) to a vertebra, said hook member (14) being fixed to said connecting body (12),
said connecting body (12) comprising:
- a first connecting member (16), and
- a second connecting member (18),
wherein the first connecting member (16) has a slot (20) adapted to receive said connecting rod (66), said slot (20) opening into two opposite side faces (22, 24) of the first connecting member (16),
**characterized in that**
the stabilizing system further comprises
- a fixing element (76) for fixing the second end (74) of said separate rod (64) to a vertebra, this fixing element (76) being distinct from said fixing devices;
and **in that** the second connecting member (18) is adapted to receive the first end (62) of said separate rod (64), in a manner that the direction of the separate rod (64) is adaptable with respect to the direction of the connecting rod (66).

2. The stabilizing system of claim 1, wherein the second connecting member (18) is provided with an opening (40) which opens into at least one side face (42) of the second connecting member (18), said opening (40) forming a socket part of a ball-and-socket joint and being configured to receive a ball end of a separate rod (64).

3. The stabilizing system of claim 2, wherein said opening (40) has a partially spherical bottom wall (44) which delimits said socket part.

4. The stabilizing system of any one of claims 1 to 3, wherein said opening (40) opens into two opposite side faces of the second connecting member (18).

5. The stabilizing system of any one of claims 1 to 4, wherein said hook member (14) and said connecting body (12) have a common median plane (P); said first connecting member (16) has an axis (XX'); and said second connecting member (18) has an axis (YY') contained in said median plane and substantially parallel to the axis (XX') of the first connecting member (18) and being offset with respect to said first connecting member in a direction perpendicular to the axis (XX') of said first connecting member.

6. The stabilizing system of claim 5, wherein said first connecting member (16) overlaps said hook member in a direction which is substantially parallel to the axis (XX') of the first connecting member (16).

7. The stabilizing system of any one of claims 1 to 6, wherein the connecting rod (66) is designed so that more than two vertebrae are stabilized by the spinal construction assembly.

8. The stabilizing system of any one of claims 1 to 7, wherein the first end (62) of the separate rod (64) forms a ball member of a ball-and-socket joint.

9. The stabilizing system of any one of claims 1 to 8, wherein said fixing element (76) includes a hook member which is, in particular, a lamina hook member or a pedicle hook member.

10. The stabilizing system of any one of claims 1 to 9, wherein said separate rod (64) is smaller in length than said connecting rod (66), said separate rod (64) being designed so that said interconnecting device (10) and said fixing element (76) are mounted on the same vertebra or on two adjacent vertebrae.

11. The stabilizing system of any one of claims 1 to 10 for interconnecting a free end of the connecting rod (66) with the separate rod (64), the slot (20) of the first connecting member (16) being adapted to receive the free end of the connecting rod (66).

12. The stabilizing system of any one of claims 1 to 11, wherein the second connecting member (18) and the first end (62) of the separate rod (64) are connected via a ball-and-socket joint.

13. The stabilizing system of claim 12, wherein the second connecting member (18) comprises a socket part and wherein the first end (62) of the separate rod (64) has a ball shape, thereby forming a ball member, the socket part cooperating with the ball member for forming said ball-and-socket joint.

## Patentansprüche

1. Stabilisierungssystem zum Stabilisieren von Wirbeln, umfassend:
- eine Spinalkonstruktionsanordnung, umfassend einen Verbindungsstab (66) und mehrere Fixierungsvorrichtungen, um Abschnitte des Verbindungsstabes (66) mit den zu stabilisierenden Wirbeln sicher zu verbinden,
wobei das Stabilisierungssystem ferner umfaßt:
- einen separaten Stab (64) mit ersten und zweiten Enden (62, 74),
- eine Verbindungsvorrichtung (10) zum Verbinden des Verbindungsstabes (66) mit dem ersten Ende (62) des separaten Stabes (64) und Befestigen dieser Stäbe (64, 66) an einem Wirbel, wobei die Verbindungsvorrichtung umfaßt:
- einen Verbindungskörper (12) und
- ein Hakenelement (14) zum Sichern des Verbindungskörpers (12) an einem Wirbel, wobei das Hakenelement (14) an dem Verbindungskörper (12) fixiert ist,
wobei der Verbindungskörper (12) umfaßt:
- ein erstes Verbindungselement (16) und
- ein zweites Verbindungselement (18),
wobei das erste Verbindungselement (16) einen Schlitz (20) aufweist, der dazu ausgelegt ist, den Verbindungsstab (66) aufzunehmen, wobei der Schlitz (20) sich zwischen zwei gegenüberliegenden Seitenflächen (22, 24) des ersten Verbindungselementes (16) öffnet,
**dadurch gekennzeichnet, daß**
das Stabilisierungssystem ferner ein Fixierelement (76) zum Fixieren des zweiten Endes (74) des separaten Stabes (64) an einem Wirbel umfaßt, wobei dieses Fixierelement (76) von den Fixiervorrichtungen verschieden ist, und daß
das zweite Verbindungselement (18) dazu ausgelegt ist, das erste Ende (62) des separaten Stabes (64) derart aufzunehmen, daß die Richtung des separaten Stabes (64) in Bezug zur Richtung des Verbindungsstabes (66) anpaßbar ist.

2. Stabilisierungssystem nach Anspruch 1, wobei das zweite Verbindungselement (18) mit einer Öffnung (40) versehen ist, die sich in wenigstens einer Seitenfläche (42) des zweiten Verbindungselementes (18) öffnet, wobei die Öffnung (40) einen Pfannenteil eines Kugelgelenkes ausbildet und dazu ausgelegt ist, ein Kugelende eines separaten Stabes (64) aufzunehmen.

3. Stabilisierungssystem nach Anspruch 2, wobei die Öffnung (40) eine zum Teil kugelförmige Bodenwand (44) aufweist, die den Pfannenteil begrenzt.

4. Stabilisierungssystem nach einem der Ansprüche 1 bis 3, wobei die Öffnung (40) sich zwischen zwei gegenüberliegenden Seitenflächen des zweiten Verbindungselementes (18) eröffnet.

5. Stabilisierungssystem nach einem der Ansprüche 1 bis 4, wobei das Hakenelement (14) und der Verbindungskörper (12) eine gemeinsame Medianebene (P) aufweisen, das erste Verbindungselement (16) eine Achse (XX') aufweist und das zweite Verbindungselement (18) eine Achse (YY') aufweist, die in der Medianebene enthalten ist und im wesentlichen parallel zur Achse (XX') des ersten Verbindungselementes (18) ist und in Bezug zum ersten Verbindungselement in einer Richtung, die senkrecht zur Achse (XX') des ersten Verbindungselementes ist, versetzt ist.

6. Stabilisierungssystem nach Anspruch 5, wobei das erste Verbindungselement (16) das Hakenelement in einer Richtung überlappt, die im wesentlichen parallel zur Achse (XX') des ersten Verbindungselementes (16) ist.

7. Stabilisierungssystem nach einem der Ansprüche 1 bis 6, wobei der Verbindungsstab (66) so ausgestaltet ist, daß er mehr als zwei Wirbel durch die Spinalkonstruktionsanordnung stabilisiert.

8. Stabilisierungssystem nach einem der Ansprüche 1 bis 7, wobei das erste Ende (62) des separaten Stabes (64) ein Kugelelement eines Kugelgelenkes bildet.

9. Stabilisierungssystem nach einem der Ansprüche 1 bis 8, wobei das Fixierelement (76) ein Hakenelement umfaßt, daß insbesondere ein Lamellenhakenelement oder ein Pedikelhakenelement ist.

10. Stabilisierungssystem nach einem der Ansprüche 1 bis 9, wobei der separate Stab (64) kürzer als der Verbindungsstab (66) ist, wobei der separate Stab (64) dazu ausgestaltet ist, daß die Verbindungsvorrichtung (10) und das Fixierelement (76) an demselben Wirbel oder an zwei benachbarten Wirbeln angebracht werden.

11. Stabilisierungssystem nach einem der Ansprüche 1 bis 10 zum Verbinden eines freien Endes des Verbindungsstabes (66) mit dem separaten Stab (64), wobei der Schlitz (20) des ersten Verbindungselementes (16) dazu ausgelegt ist, das freie Ende des Verbindungsstabes (66) aufzunehmen.

12. Stabilisierungssystem nach einem der Ansprüche 1 bis 11, wobei das zweite Verbindungselement (18) und das erste Ende (62) des separaten Stabes (64) mittels eines Kugelgelenkes verbunden werden.

13. Stabilisierungssystem nach Anspruch 12, wobei das zweite Verbindungselement (18) einen Pfannenteil umfaßt und wobei das erste Ende (62) des separaten Stabes (64) eine Kugelform aufweist, wodurch es ein Kugelelement bildet, wobei das Pfannenteil mit dem Kugelelement kooperiert, um das Kugelelement zu bilden.

## Revendications

1. Système stabilisateur pour stabiliser des vertèbres comprenant :
- un ensemble de construction vertébrale comprenant une tige de raccordement (66) et une pluralité de dispositifs de fixation pour fixer les parties de la tige de raccordement (66) avec les vertèbres à stabiliser ;
le système stabilisateur comprenant en outre :
- une tige séparée (64) ayant des première et seconde extrémités (62, 74) ; et
- un dispositif d'interconnexion (10) pour interconnecter ladite tige de raccordement (66) avec la première extrémité (62) de ladite tige séparée (64) et fixer ces tiges (64, 66) à une vertèbre, ce dispositif d'interconnexion comprenant :
- un corps de raccordement (12), et
- un crochet (14) pour fixer ledit corps de raccordement (12) à une vertèbre, ledit crochet (14) étant fixé audit corps de raccordement (12),
ledit corps de raccordement (12) comprenant :
- un premier élément de raccordement (16), et
- un second élément de raccordement (18),
dans lequel le premier élément de raccordement (16) a une fente (20) adaptée pour recevoir ladite tige de raccordement (66), ladite fente (20) débouchant sur deux faces latérales opposées (22, 24) du premier élément de raccordement (16), **caractérisé en ce que** le système stabilisateur comprend en outre un élément de fixation (76) pour fixer la seconde extrémité (74) de ladite tige séparée (64) sur une vertèbre, cet élément de fixation (76) étant différent desdits dispositifs de fixation ; et **en ce que** le second élément de raccordement (18) est adapté pour recevoir la première extrémité (62) de ladite tige séparée (64), de sorte que la direction de la tige séparée (64) est adaptable par rapport à la direction de la tige de raccordement (66).

2. Système stabilisateur selon la revendication 1, dans lequel le second élément de raccordement (18) est prévu avec une ouverture (40) qui débouche sur au moins une face latérale (42) du second élément de raccordement (18), ladite ouverture (40) formant une partie de rotule d'une articulation rotulante et étant configurée pour recevoir une extrémité sphérique d'une tige séparée (64).

3. Système stabilisateur selon la revendication 2, dans lequel ladite ouverture (40) a une paroi inférieure partiellement sphérique (44) qui délimite ladite partie de rotule.

4. Système stabilisateur selon l'une quelconque des revendications 1 à 3, dans lequel ladite ouverture (40) débouche sur deux faces latérales opposées du second élément de raccordement (18).

5. Système stabilisateur selon l'une quelconque des revendications 1 à 4, dans lequel ledit crochet (14) et ledit corps de raccordement (12) ont un plan médian commun (P) ; ledit premier élément de raccordement (16) a un axe (XX') ; et ledit second élément de raccordement (18) a un axe (YY') contenu dans ledit plan médian et sensiblement parallèle à l'axe (XX') du premier élément de raccordement (18) et étant décalé par rapport audit premier élément de raccordement dans une direction perpendiculaire à l'axe (XX') dudit premier élément de raccordement.

6. Système stabilisateur selon la revendication 5, dans lequel ledit premier élément de raccordement (16) surmonte ledit crochet suivant une direction qui est sensiblement parallèle à l'axe (XX') du premier élément de raccordement (16).

7. Système stabilisateur selon l'une quelconque des revendications 1 à 6, dans lequel la tige de raccordement (66) est conçue de sorte que plus de deux vertèbres sont stabilisées par l'ensemble de construction vertébrale.

8. Système stabilisateur selon l'une quelconque des revendications 1 à 7, dans lequel la première extrémité (62) de la tige séparée (64) forme un élément sphérique d'une articulation rotulante.

9. Système stabilisateur selon l'une quelconque des revendications 1 à 8, dans lequel ledit élément de fixation (76) comprend un crochet qui est en particulier, un crochet de lame vertébrale ou un crochet de pédicule.

10. Système stabilisateur selon l'une quelconque des revendications 1 à 9, dans lequel ladite tige séparée (64) est plus petite en longueur que ladite tige de raccordement (66), ladite tige séparée (64) étant conçue de sorte que ledit dispositif d'interconnexion (10) et ledit élément de fixation (76) sont montés sur la même vertèbre ou sur deux vertèbres adjacentes.

11. Système stabilisateur selon l'une quelconque des revendications 1 à 10, pour interconnecter une extrémité libre de la tige de raccordement (66) avec la tige séparée (64), la fente (20) du premier élément de raccordement (16) étant adaptée pour recevoir l'extrémité libre de la tige de raccordement (66).

12. Système stabilisateur selon l'une quelconque des revendications 1 à 11, dans lequel ledit second système de raccordement (18) et la première extrémité (62) de la tige séparée (64) sont raccordés via une articulation rotulante.

13. Système stabilisateur selon la revendication 12, dans lequel le second élément de raccordement (18) comprend une partie de rotule et dans lequel la première extrémité (62) de la tige séparée (64) a une forme sphérique, formant ainsi un élément sphérique, la partie de rotule coopérant avec l'élément sphérique afin de former ladite articulation rotulante.
